(19) **Europäisches Patentamt / European Patent Office / Office européen des brevets**

(11) **EP 4 412 756 B1**

(12) **FASCICULE DE BREVET EUROPEEN**

(45) Date de publication et mention de la délivrance du brevet:
**24.12.2025 Bulletin 2025/52**

(21) Numéro de dépôt: **22797425.0**

(22) Date de dépôt: **03.10.2022**

(51) Classification Internationale des Brevets (IPC):
*B01J 8/02* (2006.01)    *B01J 8/06* (2006.01)
*B01J 8/00* (2006.01)

(52) Classification Coopérative des Brevets (CPC):
**B01J 8/0214; B01J 8/009; B01J 8/0285; B01J 8/065; B01J 8/067;** B01J 2208/00159; B01J 2208/00212

(86) Numéro de dépôt international:
**PCT/FR2022/051863**

(87) Numéro de publication internationale:
**WO 2023/057711 (13.04.2023 Gazette 2023/15)**

(54) **RÉACTEUR TUBULAIRE À LIT FIXE COMPORTANT UNE MEMBRANE SÉPARATIVE**

FESTBETT-ROHRREAKTOR MIT EINER TRENNMEMBRAN

FIXED-BED TUBULAR REACTOR COMPRISING A SEPARATIVE MEMBRANE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **05.10.2021 FR 2110519**

(43) Date de publication de la demande:
**14.08.2024 Bulletin 2024/33**

(73) Titulaire: **Commissariat à l'Energie Atomique et aux Energies Alternatives**
**75015 Paris (FR)**

(72) Inventeurs:
• **DUCROS, Frédéric**
  **38054 GRENOBLE CEDEX 09 (FR)**
• **CHAMPON, Isabelle**
  **38054 GRENOBLE CEDEX 09 (FR)**

(74) Mandataire: **Santarelli**
**Tour Trinity**
**1 bis Place de la Défense**
**92400 Courbevoie (FR)**

(56) Documents cités:
**EP-A1- 0 560 157    FR-A1- 3 103 714**
**US-B2- 8 961 909**

## Description

### DOMAINE TECHNIQUE

La présente invention se rapporte au domaine général des réacteurs échangeurs, et plus particulièrement au domaine des réacteurs échangeurs catalytiques utilisant un catalyseur solide, notamment sous forme de poudre, et destinés à la mise en œuvre de réactions catalytiques endothermiques ou principalement exothermiques.

De telles réactions peuvent notamment être mises en œuvre pour la synthèse de carburants et de combustibles, par exemple des combustibles liquides, tels que le méthanol (MeOH), ou gazeux, tels que le méthane ou substitut de gaz naturel (SNG pour « Synthetic Natural Gas » en anglais), le diméthyléther (DME), les hydrocarbures ou les oléfines, obtenus à partir d'hydrogène et d'oxydes de carbone ou à partir de gaz de synthèse comprenant un mélange d'hydrogène et d'oxydes de carbone. Elles peuvent encore concerner la génération d'hydrogène à partir de méthane, la synthèse d'ammoniac à partir d'hydrogène et d'azote, ou encore les réactions d'hydrogénation et déshydrogénation liées entre l'hydrogène et des molécules de type transporteurs d'hydrogène organique liquide (ou LOHC pour « Liquid Organic Hydrogen Carriers » en anglais).

L'invention peut ainsi être tout particulièrement appliquée à des réactions très exothermiques, telles que celle de méthanation (ou plus généralement la production d'hydrocarbures ou les réactions d'hydrogénation) du monoxyde ou du dioxyde de carbone en présence d'hydrogène. Elle peut également s'appliquer à des réactions du type de celle de Fischer-Tropsch, de celle de reformage humide ou à sec du méthane ou d'autres hydrocarbures, ou encore des réactions d'oxydation ou de déshydrogénation. L'invention peut également être utilisée en tant qu'échangeur de chaleur pour des applications, notamment celles mettant en œuvre un gaz, qui nécessitent des opérations de maintenance fréquentes, par exemple dues à la corrosion, à l'encrassement, entre autres.

L'invention propose ainsi un réacteur tubulaire à lit fixe susceptible de mettre en œuvre notamment des procédés de synthèses organiques exothermiques ou endothermiques.

### ÉTAT DE LA TECHNIQUE ANTÉRIEURE

Les réacteurs catalytiques utilisant des catalyseurs solides sont largement mis en œuvre pour la synthèse de composés organiques tels que les carburants de synthèse ou les combustibles parmi lesquels on peut citer les substituts de gaz naturel (SNG), le diméthyléther, le méthanol, les hydrocarbures ou encore les oléfines.

Dans le cadre de la production d'hydrocarbures, ou plus généralement pour les réactions d'hydrogénation, à partir d'hydrogène et d'oxyde de carbone, les équilibres en jeu, ou appelés encore réactions principales par la suite, sont de manière générique les suivantes :

$$\text{Eq.1}: n\,CO + (2n+1)\,H_2 \Leftrightarrow C_nH_{2n+2} + n\,H_2O$$

$$\text{Eq.2}: n\,CO_2 + (3n+1)\,H_2 \Leftrightarrow C_nH_{2n+2} + 2n\,H_2O$$

$$\text{Eq.3}: X + n\,H_2 \Leftrightarrow (-XH_{2n}-)$$

L'équilibre décrit par la troisième équation (Eq.3) concerne les molécules de type transporteurs d'hydrogène organique liquide (ou LOHC pour « Liquid Organic Hydrogen Carriers » en anglais). C'est une réaction réversible qui est donc mise en œuvre successivement dans des séquences exothermiques puis endothermiques. Ces diverses réactions sont largement documentées sur le plan thermochimique. Elles donnent potentiellement lieu à des réactions annexes (par exemple de type WGS (« Water Gas Shift » en anglais ou réaction de changement gaz-eau), RWGS (Reverse Water Gas Shift » en anglais ou réaction inverse de changement gaz-eau), formation de Boudouard, etc.) connues de l'homme du métier et non décrites ici.

Les espèces concernées par ces réactions principales et les réactions annexes sont appelées « réactants » pour les espèces en entrée du réacteur, et « produits » pour les espèces produites par les réactions principales et annexes. De plus, « fluides réactifs » qualifie l'ensemble des espèces concernées par ces réactions. Ces réactions sont toutes associées à des conditions thermodynamiques, pression et température, préférentielles compte-tenu des performances visées : il est généralement intéressant de travailler à pression élevée et il est aussi nécessaire de correctement gérer l'exothermicité de ces réactions en extrayant de la chaleur du domaine réactionnel, ce qui permet de garantir le taux de conversion, la sélectivité, la durée de vie des catalyseurs, entre autres. D'autres fonctionnalités discutées par la suite sont aussi d'intérêt.

On connaît déjà de nombreuses architectures de réacteurs catalytiques utilisés dans l'industrie pour assurer le contrôle et le pilotage thermique de réactions chimiques endothermiques ou exothermiques. Les principaux types de réacteurs échangeurs connus pour les réactions exothermiques sont décrits ci-après.

Tout d'abord, la technologie la plus simple de réacteurs catalytiques est la technologie de réacteurs dits « à lit fixe ». Dans des réacteurs à lit fixe adiabatiques placés en cascade, l'exothermicité est alors généralement gérée par une dilution des réactifs en entrée du premier étage de réaction, par exemple par recirculation des produits, et par la mise en place d'échangeurs de chaleur destinés à refroidir le mélange réactifs-produits entre les différents réacteurs. Cette architecture présente l'avantage de la simplicité de fabrication mais nécessite la mise en place de recycles des gaz pour limiter l'élévation de température et impose l'utilisation de catalyseurs stables à haute température. Ces réacteurs sont plutôt employés comme unités centralisées fonctionnant en régime établi.

Il existe également la technologie des réacteurs échangeurs à lits fluidisés. De tels réacteurs ont été développés pour répondre au problème de transfert thermique dans les lits fixes. Ces réacteurs offrent l'avantage d'une bonne homogénéité thermique dans le réacteur, ce qui évite les points chauds mais nécessitent, à puissance équivalente, un volume de réacteur plus important que dans le cas des lits fixes à écoulement piston. Dans ces réacteurs, le catalyseur est sous forme de particules fines dont l'attrition doit être maitrisée. De plus, la fluidisation des particules impose de limiter les plages de variation de débit de gaz, ce qui rend ces réacteurs peu flexibles vis-à-vis des fonctionnements intermittents.

Une autre technologie de réacteurs échangeurs concerne les réacteurs échangeurs dans lesquels la réaction chimique a lieu au sein d'un canal réactif refroidi continument par un fluide caloporteur extérieur. La plupart de ces réacteurs sont de type tube-calandre, la réaction se produisant dans les tubes réactionnels refroidis en périphérie par un bain caloporteur. Les gaz réactifs circulent axialement dans les tubes qui contiennent un catalyseur, par exemple en poudre.

La combinaison de réacteurs échangeurs du même type ou de types différents au sein d'une même unité peut également être envisagée afin d'améliorer la conversion, la flexibilité ou la valorisation de la chaleur récupérée.

La gestion des contraintes thermiques s'exerçant sur les réacteurs commencent par la réponse au besoin de contrôle thermique. Celle-ci peut emprunter diverses voies comme exposé dans les solutions technologiques précédentes, à savoir des solutions hors réacteur telles que l'étagement de la conversion globale, avec refroidissement et/ou dilution, et/ou condensation intermédiaires ; des solutions dans le réacteur telles que l'évolution vers la notion de réacteurs échangeurs, l'intensification des échanges thermiques, la diminution des tailles des canaux réactifs (millistructuration), l'intégration de structures conductrices 3D pour homogénéisation thermique, l'étagement des injections de réactifs permettant de répartir le dépôt d'énergie.

Quant à la question de la durée de vie des catalyseurs, il faut noter que les réactions exothermiques générant une grande quantité de chaleur, ceci peut conduire à l'apparition de points chauds ayant pour conséquence la dégradation locale du catalyseur et la dégradation des performances de conversion de l'ensemble réacteur/catalyseur. Autrement dit, la dégradation du catalyseur solide peut se traduire par une désactivation de ce dernier et conduire à une réduction du taux de conversion des espèces chimiques en présence. La sélectivité des réactions mises en jeu est également affectée.

Par ailleurs, certaines réactions d'hydrogénation très utiles à l'industrie, comme la synthèse de méthanol ou la synthèse de Fischer-Tropsch, telles que décrites par les équations précédentes Eq.1 à Eq.3 sont équilibrées et ont, pour les conditions thermodynamiques retenues usuellement, des taux de conversion faibles à modérés. Une stratégie intéressante consiste alors à associer des membranes permsélectives à une ou des parois des canaux réactifs, de manière à soustraire une espèce produite, typiquement l'eau, au milieu réactif, ce qui déséquilibre la réaction et augment sa productivité. L'avantage d'un tel procédé a pu être démontré expérimentalement et théoriquement. Les innovations peuvent porter sur deux éléments principaux : les membranes et les méthodes d'intégration aux réacteurs.

Dans la solution classique de réacteur échangeur avec le catalyseur localisé dans les tubes, pour des technologies de type tube-calandre, un des problèmes est le contrôle des zones rendues plus chaudes à cause de l'exothermie de la réaction. Ce phénomène nécessite de refroidir intensément les tubes sur toutes leur surface alors qu'à un instant donné, seule une faible partie de cette surface nécessite d'être refroidie intensément, par exemple pour une injection et une circulation axiale des réactifs. Une conséquence de cela est un surdimensionnement du débit de fluide thermique.

Afin de pallier ces problèmes, il a été proposé un agencement permettant de répartir la distribution des réactifs sur toute la longueur des tubes. Cette solution permet alors d'obtenir une meilleure homogénéité de la température sur toute la longueur du réacteur. A cet égard, les documents US 3,758,279 A, US 4,374,094 A, EP 0 560 157 A1 et US 2,997,374 A proposent des réacteurs échangeurs mettant en œuvre une distribution des réactifs à partir d'un espace de distribution annulaire. Notamment, ces réacteurs échangeurs, de forme généralement cylindrique, comprennent, agencés de manière coaxiale et à partir de l'extérieur du réacteur, un tube, l'espace de distribution annulaire, une charge de catalyseur et un espace de collecte. Ces solutions constituent le standard du refroidissement dans des réacteurs de type tube et calandre, c'est-à-dire que le refroidissement des tubes est piloté uniquement par l'écoulement dans la calandre.

Ces agencements ne sont toutefois pas satisfaisants. En effet, la présence de l'espace de distribution annulaire disposé autour de la charge de catalyseur limite les transferts de chaleur du catalyseur vers le tube, rendant peu efficace les systèmes de refroidissement généralement considérés. Il reste néanmoins possible d'insérer des éléments conducteurs de chaleur dans le réacteur. Une telle solution reste toutefois incompatible avec les réacteurs comprenant des tubes de faible diamètre.

Inversement, le document CN 103990420 A propose de mettre en œuvre un insert pourvu d'une chambre de distribution et d'une chambre de collecte, disposée au centre d'un tube et définissant avec ce dernier une espace annulaire logeant le catalyseur solide. Toutefois, l'agencement proposé dans ce document ne permet pas de répartir de manière homogène au sein de l'espace annulaire. Plus particulièrement, cet agencement ne permet pas d'obtenir un profil de température optimal au sein du catalyseur solide.

La figure 1 du document US 8,961,909 A représente un autre exemple de réacteur du type tube-calandre. Ce réacteur est notamment pourvu d'un tube d'injection, plongé dans un lit de poudre catalytique, et le long duquel sont ménagés des perçages. Ces derniers sont notamment agencés pour assurer une injection du gaz réactif à différents niveaux du lit de poudre catalytique, et ainsi limiter l'apparition de points chauds dans ledit lit. Toutefois, ce réacteur n'est pas satisfaisant. En effet, afin d'assurer son refroidissement, ce réacteur requiert la mise en place d'une pluralité de circuits de circulation d'un fluide caloporteur, ce qui augmente d'autant sa complexité.

Le document US 7,402,719 B2, notamment à la figure 3a, divulgue un autre exemple de réacteur agencé pour permettre une injection étagée d'un réactif C en vue de sa réaction avec un réactif A. Ce réacteur comprend à cet égard deux nappes (ou canaux) séparées par une paroi et destinées à assurer la circulation, respectivement, du réactif A et du réactif C. Les deux nappes sont par ailleurs en communication fluidique au moyen d'une pluralité de perçages ménagés dans la paroi les séparant. Ces perçages sont notamment agencés afin d'assurer un mélange progressif du réactif C avec le réactif A. Ce mélange progressif permet ainsi de limiter l'apparition de points chauds. Cependant, l'agencement du réacteur sous forme d'un empilement de nappes rend ce dernier peu compact.

En outre, dans la demande de brevet FR 3 103 714 A1 de la Demanderesse, un dispositif de type insert organise une distribution axiale des gaz réactifs dans les tubes, une circulation des gaz dans la direction sensiblement orthoradiale à travers un lit de catalyseur dans un espace annulaire défini par la paroi interne d'un tube et la paroi externe de l'insert, et une collecte axiale des gaz produits.

Il existe encore un besoin pour améliorer ces types de réacteurs, et notamment pour doter de tels réacteurs tubes/calandre d'une circulation de fluide alternative permettant l'intégration d'un élément séparatif et la gestion du fluide permettant l'évacuation de l'espèce chimique séparée.

## EXPOSÉ DE L'INVENTION

L'invention a donc pour but de remédier au moins partiellement aux besoins mentionnés précédemment et aux inconvénients relatifs aux réalisations de l'art antérieur.

L'invention vise notamment à proposer un réacteur tubulaire à lit fixe permettant une distribution plus uniforme des réactifs au sein du catalyseur solide, une répartition plus homogène du flux de chaleur généré au sein du catalyseur solide, une meilleure gestion du refroidissement. De plus, l'invention cherche à proposer un réacteur tubulaire pour lequel la fiabilité et la durée de vie (des catalyseurs) sont améliorées au regard des réacteurs connus de l'état de la technique, et permettant d'optimiser (augmenter) le temps de passage des gaz dans le lit fixe de poudre catalytique.

L'invention a ainsi pour objet, selon l'un de ses aspects, un réacteur tubulaire à lit fixe qui s'étend, selon un axe longitudinal, entre une première extrémité et une deuxième extrémité, ledit réacteur comprenant un lit de poudre catalytique confiné dans un espace annulaire situé entre une paroi externe d'un tube creux et une paroi interne d'un insert creux disposé de manière coaxiale dans le tube creux, l'insert creux comprenant au moins une chambre de distribution et au moins une chambre de collecte, séparées l'une de l'autre par au moins une première paroi séparatrice, ladite au moins une chambre de distribution et ladite au moins une chambre de collecte comprenant, respectivement, une ouverture d'admission de gaz au niveau de la première extrémité et une ouverture d'évacuation de gaz au niveau de la deuxième extrémité,

caractérisé en ce que la paroi interne de l'insert creux est recouverte, notamment partiellement ou totalement, d'une structure séparatrice comprenant au moins une membrane permsélective permettant une élimination partielle d'au moins un produit de réaction, de sorte que l'espace annulaire soit délimité par la paroi externe et la membrane permsélective,

et en ce que le réacteur comporte en plus au moins une chambre d'appoint séparée de ladite au moins une chambre de distribution et de ladite au moins une chambre de collecte par au moins une première paroi séparatrice, ladite au moins une chambre d'appoint comprenant un orifice d'entrée d'au moins un fluide d'appoint constitué par un fluide de balayage permettant l'évacuation dudit au moins un produit de réaction, distinct des gaz circulant dans ladite au moins une chambre de distribution et dans ladite au moins une chambre de collecte, au niveau de la première extrémité et un orifice de sortie dudit au moins un fluide d'appoint au niveau de la deuxième extrémité.

Grâce à l'invention, il est notamment possible d'améliorer le refroidissement de la zone réactionnelle (réactifs, produits de réaction et catalyseurs) en permettant de refroidir cette zone par une circulation interne et externe de fluide caloporteur, ce qui est bénéfique pour l'ensemble des réactions d'hydrogénation par exemple. De plus, elle peut permettre d'améliorer le taux de conversion des réactions équilibrées telles que la synthèse de méthanol, d'oléfines, d'hydrocarbures ou encore d'hydrogénation des molécules de type transporteurs d'hydrogène organique liquide (LOHC).

Le réacteur selon l'invention peuvent en outre comporter l'une ou plusieurs des caractéristiques suivantes prises isolément ou suivant toutes combinaisons techniques possibles.

La membrane permsélective peut être organique ou inorganique. Elle peut avantageusement être conformable. Elle peut être telle que décrite dans l'ouvrage « In-Situ H2O removal via hydrophilic membranes during Fischer-Tropsch and other fuel-related synthesis reactions », M.P.Rhode, Phd Dissertation, KIT Scientific Publishing, 2010.

La perméabilité de la membrane permsélective peut avantageusement s'exercer de façon sélective vis-à-vis de la vapeur d'eau.

Selon une variante, la structure séparatrice peut comporter de plus un support poreux recouvrant la paroi interne de l'insert, le support poreux étant lui-même recouvert par la membrane permsélective, et l'insert creux peut comporter des perforations pour permettre le passage dudit au moins un produit de réaction à séparer desdits gaz.

Selon une autre variante, l'insert creux peut être réalisé en un matériau poreux, pour permettre le passage dudit au moins un produit de réaction à séparer desdits gaz, et recouvert par la membrane permsélective, et ladite au moins une première paroi séparatrice de l'insert creux peut comporter un matériau d'étanchéité, notamment céramique, pour l'étanchéité vis-à-vis des gaz réactifs.

Chacune desdites au moins une chambre de distribution, au moins une chambre de collecte et au moins une chambre d'appoint peut être délimitée par une section de la paroi interne et deux premières parois séparatrices.

De plus, le réacteur peut comporter au niveau de la première extrémité et au niveau de la deuxième extrémité, respectivement, un espace distributeur et un espace collecteur entre lesquels l'insert creux est disposé.

En outre, le tube et l'insert peuvent être maintenus par au moins deux plaques tubulaires de maintien respectivement au niveau des première et deuxième extrémités, au moins l'une desdites au moins deux plaques tubulaires de maintien, notamment toutes les plaques tubulaires de maintien, comportant un conduit d'entrée ou de sortie respectivement relié fluidiquement à un orifice d'entrée ou de sortie de fluide d'appoint et formé dans ladite au moins l'une desdites au moins deux plaques tubulaires de maintien, pour permettre notamment une admission et/ou une extraction latérale de fluide d'appoint.

Les longueurs axiales du tube et de l'insert peuvent être différentes, la longueur axiale du tube étant notamment plus courte que la longueur axiale de l'insert.

En variante, le tube et l'insert peuvent être maintenus par au moins deux plaques tubulaires de maintien respectivement au niveau des première et deuxième extrémités, et au moins un conduit d'entrée ou de sortie respectivement relié fluidiquement à un orifice d'entrée ou de sortie de fluide d'appoint peut être situé respectivement dans l'espace distributeur et dans l'espace collecteur, hors desdites au moins deux plaques tubulaires de maintien, pour permettre notamment une admission et/ou une extraction latérale de fluide d'appoint.

En variante encore, le tube et l'insert peuvent être maintenus par au moins deux plaques tubulaires de maintien respectivement au niveau des première et deuxième extrémités, et ledit orifice d'entrée et ledit orifice de sortie dudit au moins un fluide d'appoint peuvent être respectivement formés dans les extrémités supérieure et inférieure de l'insert pour permettre respectivement une alimentation depuis l'espace distributeur et une extraction depuis l'espace collecteur, le réacteur comprenant en outre au moins un conduit d'alimentation latéral de gaz et au moins un conduit d'extraction latéral de gaz.

Ledit au moins un conduit d'alimentation latéral et l'alimentation en fluide d'appoint de l'espace distributeur d'une part, et ledit au moins un conduit d'extraction latéral et l'extraction en fluide d'appoint de l'espace collecteur d'autre part, peuvent être séparés par une plaque de séparation étanche.

En outre, un joint d'étanchéité peut être disposé entre l'insert et chacune des plaques de séparation étanches.

Par ailleurs, l'écoulement dudit au moins un fluide d'appoint peut être réalisé de manière co-courante ou contre-courante à l'écoulement des gaz dans lesdites au moins une chambre de distribution et de collecte.

Le cas échéant, la poudre catalytique peut être retenue dans l'espace annulaire par un joint en matière fibreuse au niveau de chacune des extrémités de l'espace annulaire.

De plus, l'insert peut être une pièce monobloc.

Par ailleurs, l'invention a encore pour objet, selon un autre de ses objets, l'utilisation d'un réacteur tubulaire tel que défini précédemment, caractérisée en ce qu'elle met en œuvre des réactions endothermiques ou exothermiques de synthèse de carburants et de combustibles.

## BRÈVE DESCRIPTION DES DESSINS

L'invention pourra être mieux comprise à la lecture de la description détaillée qui va suivre, d'exemples de mise en œuvre non limitatifs de celle-ci, ainsi qu'à l'examen des figures, schématiques et partielles, du dessin annexé, sur lequel :

- la figure 1 est une représentation schématique partielle d'un réacteur tubulaire à lit fixe conforme à l'invention, selon un plan en coupe, passant par l'axe longitudinal du réacteur, en particulier selon le plan de coupe longitudinale PP de la figure 2, permettant de visualiser une chambre de collecte et une chambre de distribution,
- la figure 2 est une vue en coupe selon un plan transversal, ou section normale, perpendiculaire à l'axe longitudinal du réacteur tubulaire de la figure 1,
- la figure 2A est une vue semblable à celle de la figure 2, permettant de visualiser une première variante de l'utilisation d'une structure séparatrice selon le principe de l'invention,
- la figure 2B est une vue semblable à celle de la figure 2, permettant de visualiser une deuxième variante de l'utilisation d'une structure séparatrice selon le principe de l'invention,
- la figure 3 est une représentation schématique du réacteur tubulaire des figures 1 et 2 selon le plan de coupe longitudinale P'P' de la figure 2, permettant de visualiser deux chambres d'appoint, alimentées par des orifices d'entrée et de sortie latéraux,
- la figure 4 est une vue semblable à celle de la figure 1 permettant d'illustrer l'utilisation de joints pour retenir le catalyseur,
- la figure 5 est une vue semblable à celle de la figure 3 permettant d'illustrer l'utilisation de joints pour retenir le catalyseur,
- la figure 6 est une représentation schématique d'un autre exemple de réacteur tubulaire conforme à l'invention, selon une vue semblable à celle de la figure 1,
- la figure 7 est une vue semblable à celle de la figure 3 pour le réacteur de la figure 6,
- la figure 8 est encore une représentation schématique d'un autre exemple de réacteur tubulaire conforme à l'invention, selon une vue semblable à celle de la figure 1,
- la figure 9 est une vue semblable à celle de la figure 3 pour le réacteur de la figure 8,
- les figures 10 et 11 sont des vues, en coupe axiale respectivement selon les plans PP et P'P' en référence à la figure 2, d'une pluralité de réacteurs tubulaires semblables à celui des figures 1 à 5, situés à l'intérieur d'une calandre,
- la figure 12 est une vue, en coupe axiale selon le plan P'P' en référence à la figure 2, d'une pluralité de réacteurs tubulaires semblables à celui des figures 6 et 7, situés à l'intérieur d'une calandre,
- la figure 13 est un graphique représentant l'évolution de la conversion de dioxyde de carbone en fonction de la longueur d'arc de l'insert, dans une configuration avec présence de membrane et dans une configuration sans la présence de membrane, et
- la figure 14 est un graphique représentant une coupe dans le catalyseur montrant l'évolution des concentrations molaires et les lignes de flux total d'eau ($H_2O$).

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

## EXPOSÉ DÉTAILLÉ DE MODES DE RÉALISATION PARTICULIERS

La présente invention concerne un réacteur échangeur tubulaire à lit de poudre catalytique fixe. En particulier, le lit de poudre catalytique est confiné dans un espace annulaire situé entre une paroi, dite paroi externe, d'un tube creux et une autre paroi, dite paroi interne, d'un insert creux logé de manière coaxiale dans ledit tube. Le lit de poudre catalytique peut notamment comporter un catalyseur sous forme de grains.

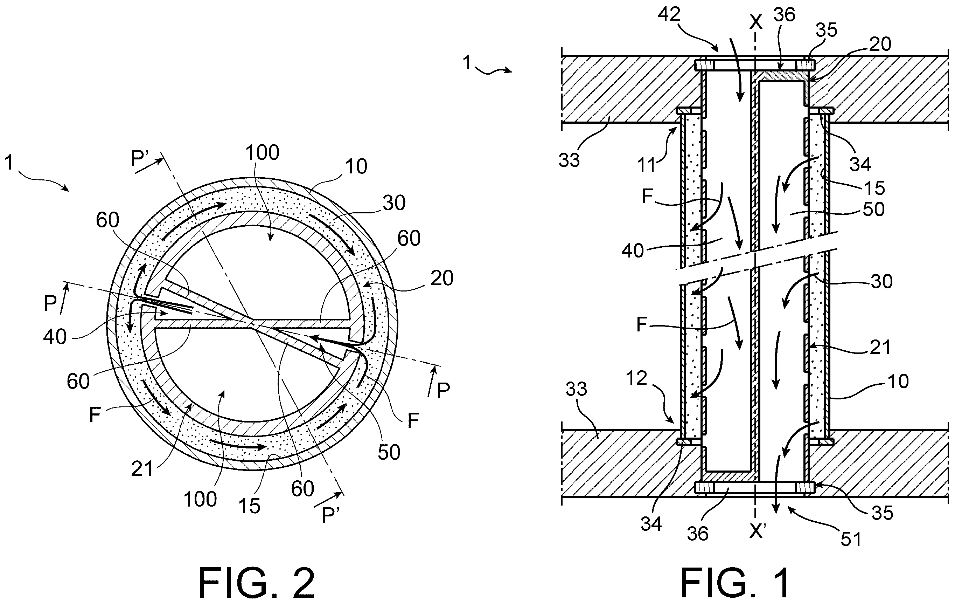

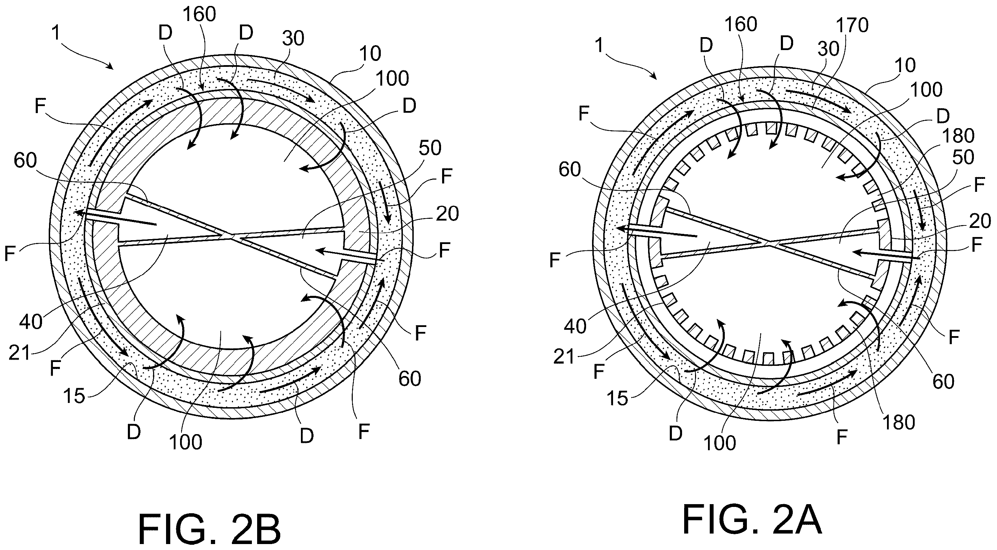

Il est à noter que sur l'ensemble des figures 1, 2 et 3 à 12, la structure séparatrice comprenant une membrane permsélective 160 selon le principe de l'invention n'est pas représentée. Il convient donc de se référer aux figures 2A et 2B qui illustrent ce principe. Aussi, il faut noter que les variantes de structure séparatrice décrites en référence aux figures 2A et 2B sont applicables aux modes de réalisation des figures 1, 2 et 3 à 12.

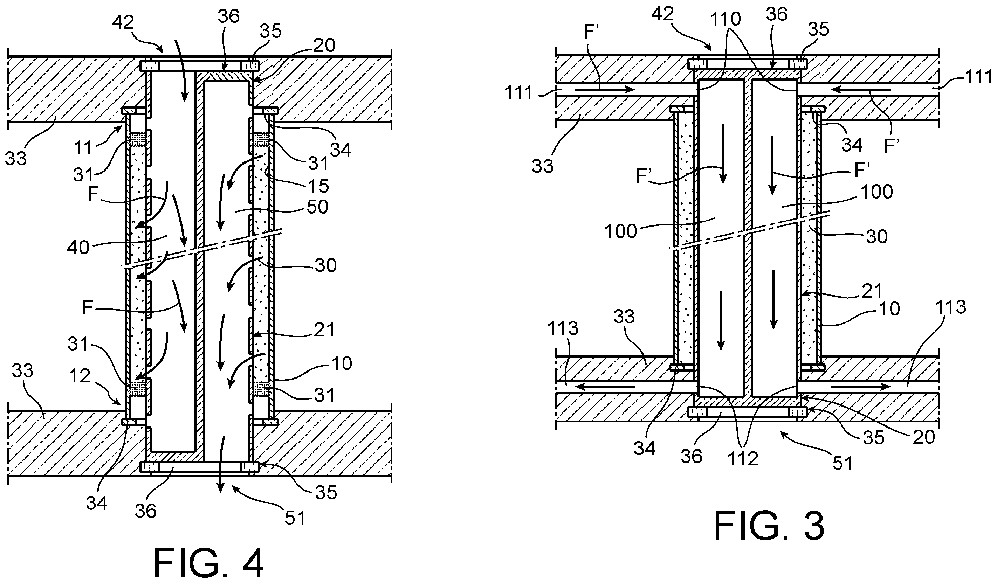

Ainsi, aux figures 1 et 2, on peut voir un exemple de réalisation d'un réacteur tubulaire à lit fixe selon la présente invention. Il est à noter que sur ces figures 1 et 2, ainsi que sur l'ensemble des figures décrites par la suite, les flèches F représentent le sens de parcours du gaz. Par ailleurs, les flèches F', notamment visibles sur les figures 3, 5, 7, 9 et 11, représentent le sens de parcours du fluide d'appoint.

Le réacteur tubulaire 1 selon la présente invention comprend un tube creux 10 externe qui s'étend selon un axe longitudinal XX' entre une première extrémité 11 et une deuxième extrémité 12. Le tube creux 10 peut présenter une symétrie de révolution autour de l'axe longitudinal XX'. Il est donc entendu que l'axe longitudinal XX' peut être un axe de révolution du tube creux 10.

Le tube creux 10 peut comprendre un métal, et notamment un métal choisi parmi : acier, alliage d'aluminium, de cuivre, de nickel, entre autres. Le diamètre du tube creux 10 peut être compris entre 5 mm et 100 mm. La paroi, dite paroi externe 15, formant le tube creux 10 peut présenter une épaisseur comprise entre 0,5 mm et 10 mm. Le tube creux 10 peut présenter une longueur comprise entre 10 fois et 200 fois le diamètre de la surface interne.

Le réacteur tubulaire 1 comprend également un insert creux 20 qui s'étend également selon l'axe longitudinal XX' et présente une forme généralement cylindrique. L'insert creux 20 est notamment logé dans le volume du tube creux 10 de manière coaxiale à ce dernier. En particulier, l'insert 20 comprend également une paroi, dite paroi interne 21, en particulier une paroi perméable au gaz, qui délimite avec la paroi externe 15 un espace annulaire 30. L'espace annulaire 30 est, à cet égard, rempli d'une poudre catalytique, et sera le siège des réactions de conversion de gaz réactifs susceptibles de transiter dans le réacteur tubulaire 1. L'espace annulaire 30 peut présenter une épaisseur, définie comme la distance entre la paroi externe 15 et la paroi interne 21, comprise entre 2% et 20 % du diamètre de la surface interne du tube creux 10. L'insert creux 20 peut être une pièce monobloc. L'insert creux 20 peut par exemple être réalisé en acier inoxydable, notamment par des méthodes de brasage, ou en aluminium, notamment par des méthodes d'extrusion ou de fabrication additive (par exemple un procédé de fabrication 3D), voire en polymères pour certaines réactions à faible température. Les diverses ouvertures de l'insert creux 20 peuvent être réalisées lors de la fabrication de l'insert, et/ou réalisées par usinage dans un second temps.

L'insert creux 20 comprend par ailleurs au moins une chambre de distribution 40 et au moins une chambre de collecte 50. Ici, par souci de simplicité, une seule chambre de distribution 40 avec un seul point d'injection et une seule chambre de collecte 50 sont représentées mais ce choix n'est pas limitatif. En particulier, l'insert creux 20 peut comprendre notamment entre 1 et 4 chambres de distribution 40, et entre 1 et 4 chambres de collecte 50.

Ladite au moins une chambre de distribution 40 et ladite moins une chambre de collecte 50 sont avantageusement disposées en alternance, et s'étendent sur toute la longueur de l'insert creux 20.

Avantageusement, l'insert creux 20 comprend encore au moins une chambre d'appoint 100 organisant la circulation axiale d'un fluide d'appoint, ici deux chambres d'appoint 100, mais l'invention n'est pas limitée sur le nombre de chambres et de fluides d'appoint supplémentaires.

Ainsi, on trouve successivement par observation d'un plan en section normale à l'axe XX' du tube creux 10, comme visible sur la figure 2, une première chambre d'appoint 100, ladite au moins une chambre de collecte 50, une deuxième chambre d'appoint 100 et ladite au moins une chambre de distribution 40. Ladite au moins une chambre de collecte 50, ladite au moins une chambre de distribution 40 et les chambres d'appoint 100 sont par ailleurs séparées les unes des autres par des premières parois séparatrices 60. Il est donc entendu qu'une chambre de distribution 40 est délimitée par deux parois séparatrices 60 et une section de la paroi interne 21. De manière équivalente, une chambre de collecte 50 est également délimitée par deux premières parois séparatrices 60 et une autre section de la paroi interne 21. De manière encore équivalente, une chambre d'appoint 100 est séparée par deux premières parois séparatrices 60 et encore un autre section de la paroi interne 21.

En outre, les premières parois séparatrices 60 s'étendent sur toute la longueur de l'insert creux 20 dans le volume défini par le tube creux 10, et sont agencées pour prévenir tout passage direct de gaz d'une chambre à l'autre. Par exemple, les premières parois séparatrices 60 forment des plans passant par l'axe longitudinal XX'.

Notamment, les deux premières parois séparatrices 60 d'une chambre de distribution 40 peuvent présenter une forme généralement allongée et s'étendre selon l'axe longitudinal XX' de la première extrémité 11 vers la deuxième extrémité 12. En particulier, les deux premières parois séparatrices 60 d'une chambre de distribution 40 peuvent présenter un côté commun en coïncidence avec l'axe longitudinal XX'.

En outre, le réacteur 1 peut comporter, au niveau de la première extrémité 11 de l'insert creux 20, un espace distributeur 42, ou plenum d'entrée, par lequel un ou des gaz réactifs sont susceptibles d'être admis dans la chambre de distribution 40 au travers d'une ouverture d'admission. De même, le réacteur 1 peut comporter, au niveau de la deuxième extrémité 12 de l'insert creux 20, un espace collecteur 51, ou plenum de sortie, par lequel un ou plusieurs gaz sont susceptibles d'être évacués au travers d'une ouverture d'évacuation.

Par ailleurs, la chambre de distribution 40 est obturée au niveau de la deuxième extrémité 12, et la chambre de collecte 50 est obturée au niveau de la première extrémité 11.

La paroi interne 21 peut en outre comprendre au moins une ouverture distributrice et au moins une ouverture collectrice, permettant respectivement la distribution d'un gaz susceptible d'être admis par l'ouverture d'admission au niveau du plenum d'entrée 42 dans un compartiment de distribution vers l'espace annulaire 30, et la collecte du gaz distribué dans l'espace annulaire 30 par la chambre de collecte 50.

Le tube creux 10 est avantageusement maintenu par deux plaques tubulaires de maintien 33 lesquelles comportent chacune un premier système de fixation étanche 34 du tube creux 10 à chaque plaque de maintien 33. De même, l'insert creux 20 est avantageusement maintenu par les deux plaques de maintien 33 lesquelles comportent chacune un deuxième système de fixation étanché 35 de l'insert creux 20 à chaque plaque de maintient 33. Des parois de séparation 36 sont également présentes dans chacune des plaques de maintien 33, entre lesquelles sont contenus le tube creux 10 et l'insert creux 20.

Conformément à l'invention, et comme visible sur les figures 2A et 2B, la paroi interne 21 de l'insert creux 20 est recouverte, totalement ou partiellement, par une structure séparatrice 160, 170 comprenant une membrane permsélective 160 permettant une élimination partielle d'un produit de réaction et une amélioration de la productivité de la réaction chimique, de sorte que l'espace annulaire 30 soit délimité par la paroi externe 15 et la membrane permsélective 160.

De façon avantageuse mais non limitative, la perméabilité de la membrane permsélective 160 s'exerce ici de façon sélective vis-à-vis de la vapeur d'eau $H_2O$.

L'ajout d'une telle membrane permsélective 160 permet avantageusement de créer un déséquilibre de la réaction chimique profitable à la performance de l'ensemble. La membrane 160 peut être organique ou inorganique, et préférentiellement conformable.

Cette membrane 160 peut permettre de prélever une partie de la vapeur d'eau $H_2O$ en considérant que le fluide d'appoint F' est ici un fluide de balayage qui permet d'évacuer les espèces à éliminer, ici la vapeur d'eau $H_2O$. La chambre de collecte 50 comporte une concentration en vapeur d'eau $H_2O$ a priori résiduelle.

Les figures 2A et 2B représentent deux variantes de réalisation de la structure sélective permettant l'extraction du produit de réaction, ici la vapeur d'eau $H_2O$. Sur ces figures 2A et 2B, les flèches D représentent la diffusion au travers de la membrane 160.

Sur l'exemple de la figure 2A, la structure séparatrice comporte de plus un support poreux 170 recouvrant la paroi interne 21 de l'insert 20. Ce support poreux 170 est lui-même recouvert par la membrane permsélective 160. De plus, l'insert creux 20 comporte des perforations 180 pour permettre le passage de la vapeur d'eau $H_2O$.

Sur l'exemple de la figure 2B, l'insert creux 20 est réalisé en un matériau poreux, pour permettre le passage de la vapeur d'eau $H_2O$, et recouvert par la membrane permsélective 160. De plus, chaque première paroi séparatrice 60 de l'insert creux 20 comporte un matériau d'étanchéité, notamment en céramique, pour l'étanchéité vis-à-vis des gaz réactifs.

Aussi, l'invention met avantageusement à profit l'utilisation des chambres d'appoint 100 pour permettre la circulation et la collecte de la ou des espèces à séparer, ici la vapeur d'eau $H_2O$.

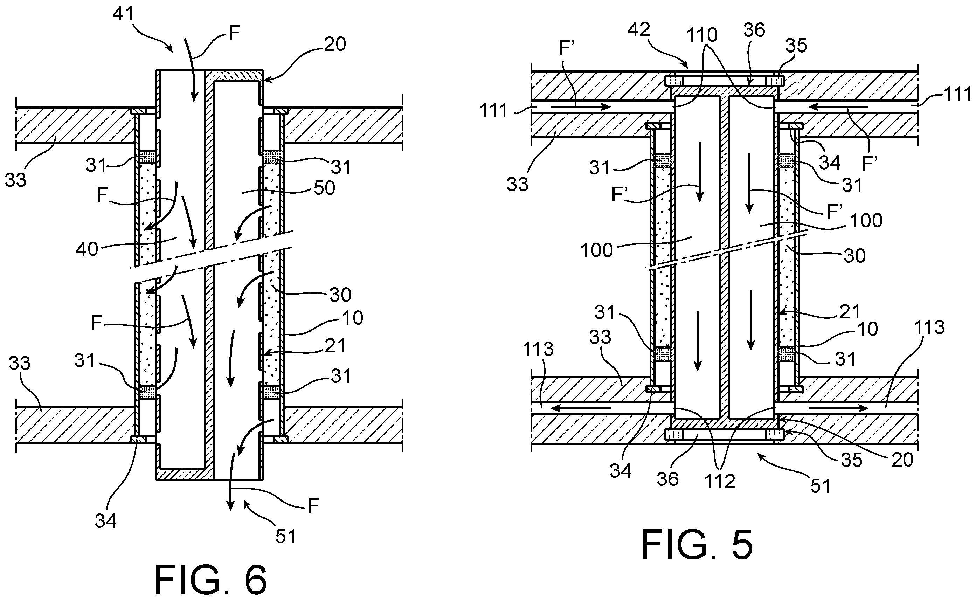

Selon un aspect avantageux illustré aux figures 4 et 5, la poudre catalytique est retenue dans l'espace annulaire 30 par un joint 31, par exemple en matière fibreuse, au niveau de chacune des extrémités de l'espace annulaire 30. Dans la mesure où le joint 31 est en matière fibreuse, ce dernier est nécessairement poreux et donc perméable aux gaz réactifs. La matière fibreuse peut à cet égard comprendre au moins un des éléments choisi parmi : fibre de verre, fibre de céramique, fibre de métal, fibre de carbone, fibre de matériau polymère.

Le joint 31 peut notamment être sous forme d'une tresse, d'une gaine, d'une cordelette ou simplement comprendre un bourrage de la matière fibreuse. La matière fibreuse est avantageusement un isolant thermique et présente une conductivité thermique sensiblement équivalente à celle du catalyseur utilisé (0,2 W/m/K à 10 W/m/K).

La figure 3 permet de visualiser, en coupe axiale selon le plan P'P' de la figure 2, les chambres d'appoint 100 décrites précédemment. Chaque chambre d'appoint 100 est alimentée par un orifice d'entrée latéral 110, situé à proximité de l'extrémité 11 du tube creux 10, cet orifice d'entrée 110 étant alimenté par un conduit d'entrée latéral 111 formé dans la plaque de maintien 33 supérieure.

De même, chaque chambre d'appoint 100 comporte un orifice de sortie latéral 112, situé à proximité de l'extrémité 12 du tube creux 10, cet orifice de sortie 112 étant relié fluidiquement à un conduit de sortie latéral 113 formé dans la plaque de maintien 33 inférieure. De cette manière, un fluide d'appoint F' peut être admis par circulation dans les plaques tubulaires de maintien 33.

De façon avantageuse, l'invention permet ainsi d'étendre les capacités de thermalisation du réacteur 1, en particulier de permettre de gérer et de faire circuler des fluides d'appoint, notamment des fluides utilitaires ou réactifs, en plus des réactifs et produits de réaction, par exemple selon les équations Eq. 1 et/ou Eq. 2 décrites précédemment.

La géométrie de l'insert creux 20 et la géométrie du tube externe creux 10 sont définies de manière à permettre une alimentation séparée des chambres de distribution 40 et d'appoint 100, et des sorties séparées des chambres de collecte 50 et d'appoint 100.

Dans l'exemple de réalisation des figures 1 à 5, le tube creux 10 et l'insert creux 20 ont des longueurs axiales différentes, le tube creux 10 étant moins long que l'insert creux 20, et l'alimentation et l'évacuation en fluide d'appoint se fait au travers d'une plaque de maintien 33, par le biais des conduits 111 et 113. De plus, l'écoulement du fluide d'appoint F' est dirigé axialement et de manière co-courante de la circulation des gaz. Toutefois, un concept à contre-courant peut également être intéressant.

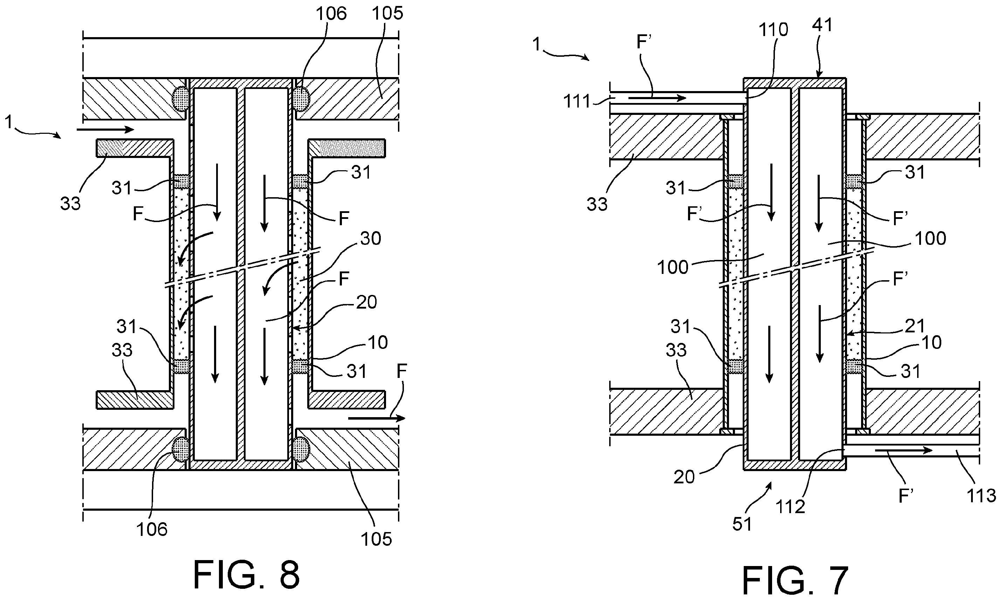

Dans l'exemple de réalisation des figures 6 et 7, l'alimentation des chambres d'appoint 100 est assurée par le biais de conduits dédiés situés dans les plenums d'entrée 42 et de sortie 51, et non plus par le biais de conduits usinés dans des plaques de maintien 33.

Plus précisément, comme visible sur la figure 7, un conduit d'entrée latéral 111, pour l'admission de fluide d'appoint F', est situé le long de la plaque de maintien 33 supérieure, hors de celle-ci, dans la partie haute du réacteur 1. De même, un conduit de sortie latéral 112, pour l'extraction de fluide d'appoint F', est situé le long de la plaque de maintien 33 inférieure, hors de celle-ci, dans la partie basse du réacteur 1.

Ainsi, les alimentations et évacuations du fluide d'appoint F' sont assurées par des conduits 111, 112 réalisés hors des plaques tubulaires de maintien 33, et assurant une liaison étanche avec l'extérieur des plenums d'entrée 41 et de sortie 51. De plus, l'écoulement du fluide d'appoint F' est dirigé axialement et de manière co-courante de la circulation des gaz. Toutefois, un concept à contre-courant peut également être intéressant.

Dans l'exemple de réalisation des figures 7 et 8, l'alimentation des chambres d'appoint 100 est assurée par le biais de piquages supplémentaires dans les plenums d'entrée 42 et de sortie 51.

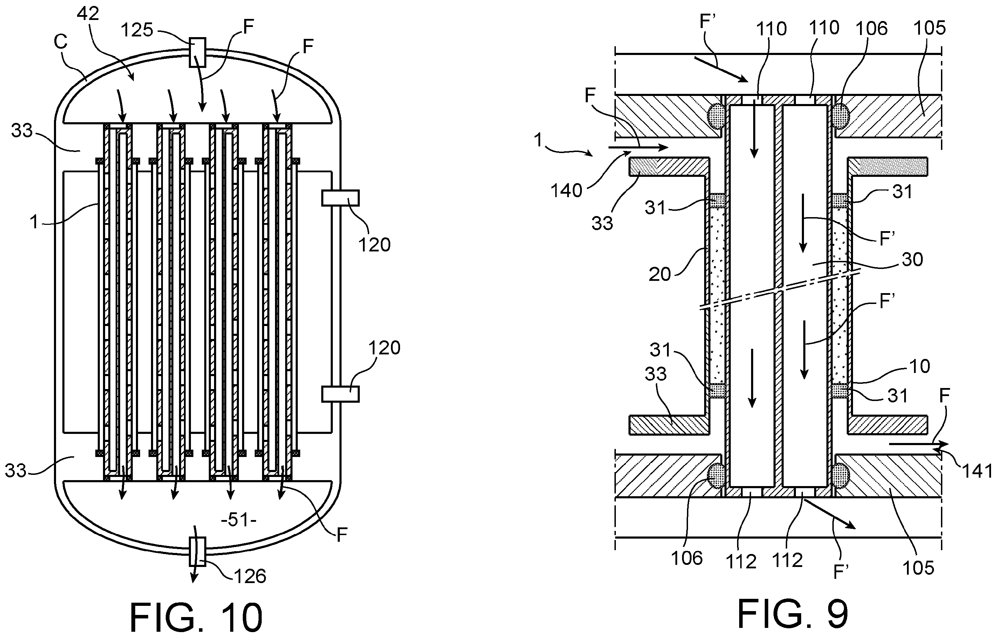

Plus précisément, comme visible sur la figure 7, l'alimentation et l'extraction en fluide d'appoint F' sont assurées par les extrémités supérieure et inférieure de l'insert creux 20. Les fluides réactifs F sont alors introduits latéralement par le biais de conduits latéraux d'admission 140 et d'extraction 141 de fluides réactifs, comme visible sur la figure 9. La séparation étanche entre les fluides d'appoint F' et les fluides réactifs F est assurée par le biais de plaques de séparation 105, démontables, introduites dans les plenums d'entrée et de sortie, et disposées chacune autour de l'insert creux 20. Un joint d'étanchéité 106 est alors placé entre chaque plaque de séparation 105 et l'insert creux 20.

De façon avantageuse, les modes de réalisation des figures 5 et 6 d'une part, et des figures 7 et 8 d'autre part, permettent de réaliser un assemblage plaque tubulaire et tubes proche des réalisations classiques.

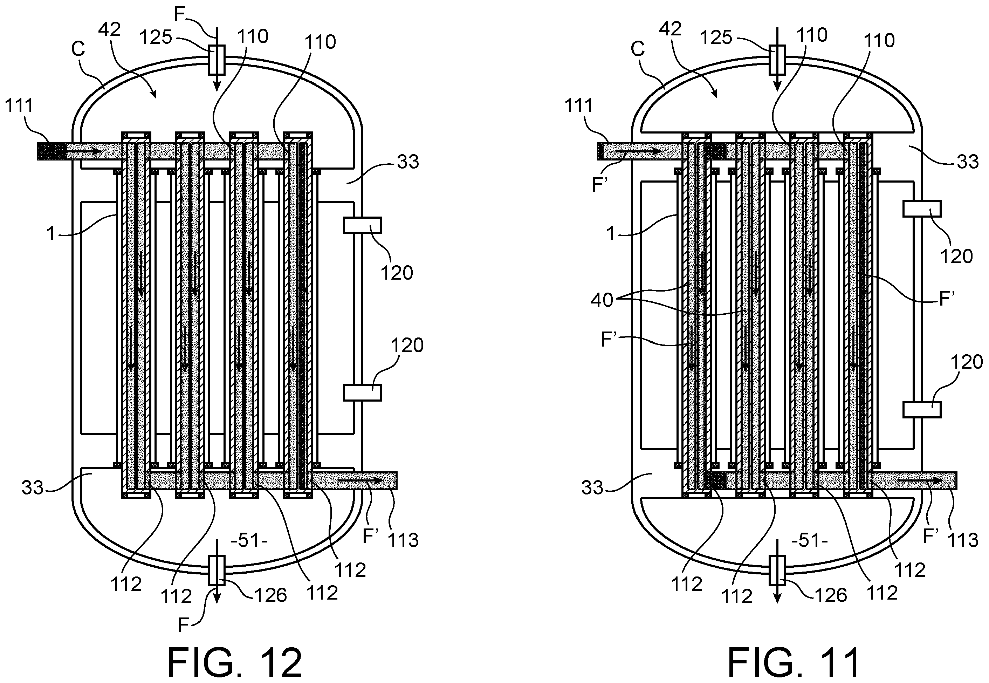

Les figures 10 et 11 sont une illustration de la mise en œuvre d'une pluralité de réacteurs tubulaires 1 conformes à l'invention, et notamment selon la variante des figures 1 à 5. De même, la figure 12 est une illustration de la mise en œuvre d'une pluralité de réacteurs tubulaires 1 conformes à l'invention selon la variante des figures 6 et 7.

Cette mise en œuvre comprend notamment quatre tubes 1 disposés parallèlement les uns aux autres dans une calandre C. Les plaques de maintien tubulaires 33 permettent de maintenir les tubes 1, et de ménager un espace de circulation d'un fluide caloporteur destiné au refroidissement des tubes 1, par le biais de systèmes d'alimentation et d'évacuation de fluide caloporteur 120.

Dans l'exemple des figures 10 et 11, la ou les chambres de distribution 40 sont alimentées directement depuis le plenum d'entrée 42 par le biais de systèmes d'alimentation en réactifs 125. Puis, l'évacuation des produits et réactifs non convertis est réalisée dans le plenum de sortie 51 par le biais de systèmes d'extraction 126.

Ici, les tubes externes sont plus courts que les inserts 20, et le fluide d'appoint F' est distribué directement dans les inserts 20 par des conduits 111, 113 intégrés aux plaques de maintien tubulaires 33.

Dans l'exemple de la figure 12, les inserts creux 20 sont plus longs. L'alimentation et l'extraction du fluide d'appoint F' sont réalisées par un circuit dédié de conduits 111, 113 situés hors des plaques de maintien 33.

Le réacteur tubulaire 1 selon la présente invention, et notamment la mise en œuvre de chambres d'appoint 100 permettant la circulation de fluides utilitaires ou réactifs, permet d'étendre les capacités de thermalisation.

Cette configuration répond de plus favorablement à la problématique d'échauffement de la poudre catalytique, notamment un catalyseur sous forme de poudres, et limite ainsi l'apparition de points chauds. Il en résulte un dispositif plus performant et plus durable. En outre, la disposition de la poudre catalytique dans l'espace annulaire 30 facilite le refroidissement de cette dernière.

Exemple d'application : application à la synthèse de méthanol (MeOH)

Deux modélisations numériques ont été réalisées sur le principe de l'invention avec utilisation d'une membrane permsélective 160 avec pour cible la synthèse directe de méthanol (MeOH) à partir de dioxyde de carbone ($CO_2$).

Seule une section de tube a été modélisée, dans une configuration cohérente avec la variante de la figure 2B. Les conditions de la simulation ont été : une pression (P) de 50 bar, et une température (T) d'entrée des réactifs de 200°C, pour des conditions d'entrée stœchiométriques pour cette réaction.

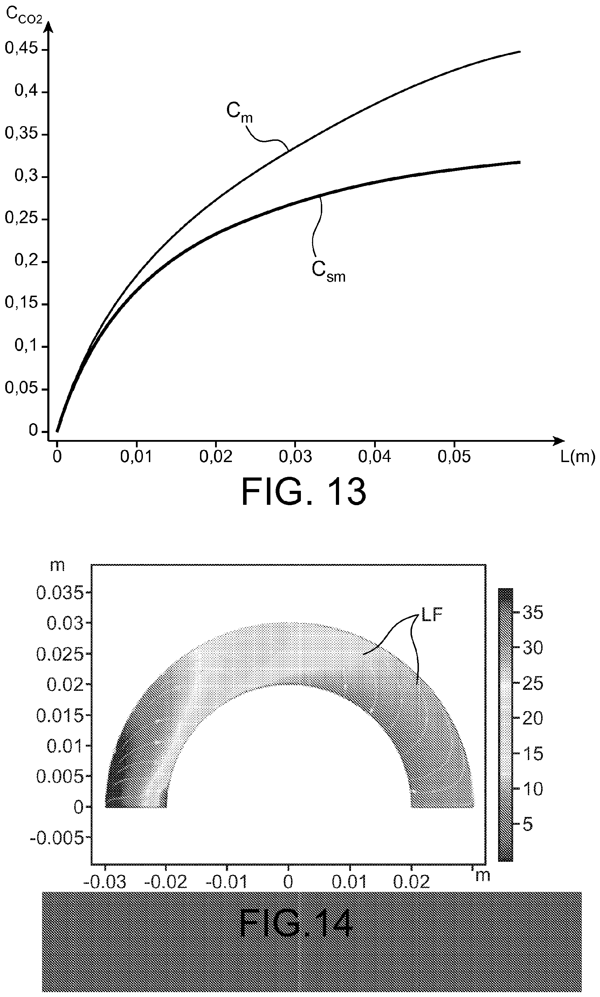

Les résultats obtenus sont notamment visibles sur le graphique de la figure 13 qui représente l'évolution de la conversion de dioxyde de carbone ($C_{CO2}$) en fonction de la longueur L, exprimée en mètres (m), longueur d'arc de l'insert 20, dans une configuration avec présence de membrane ($C_m$) et dans une configuration sans la présence de membrane ($C_{sm}$), à 200°C et à 50 bar, et sur le graphique de la figure 14 qui représente une coupe dans le catalyseur montrant l'évolution des concentrations molaires m, exprimées en mol/m$^3$, pour l'espèce $H_2O$ et les lignes LF de flux total d'eau.

Les résultats montrent un taux de conversion accru en conséquence de l'action séparatrice des membranes 160, et selon les régimes d'écoulement, un appauvrissement en concentration d'$H_2O$ dans les produits de réactions visibles sur toute l'épaisseur du catalyseur.

Bien entendu, l'invention n'est pas limitée aux exemples de réalisation qui viennent d'être décrits. Diverses modifications peuvent y être apportées par l'homme du métier.

En particulier, le nombre, et la disposition respective et angulaire, des chambres d'appoint 100, de collecte 50 et de distribution 40 peut varier.

Le sens de circulation des fluides d'appoint F' dans les chambres d'appoint 100 peut varier.

Le choix de la localisation des alimentations et extractions, notamment en extrémité d'insert 20 ou par piquage latéral de l'insert 20, respectivement des fluides d'appoint F' et de réactifs F peut varier en fonction du procédé.

L'alimentation des chambres d'appoint 100 peut ou non être associée aux plaques tubulaires 33.

Le réacteur 1 selon l'invention peut ou non comprendre des moyens d'injection étagée.

Pour le cas où les chambres d'appoint 100 sont utilisées comme chambres de refroidissement, l'efficacité du système peut encore être amélioré par diverses manipulations visant à intensifier les échanges thermiques et connues par l'homme du métier, telles que structuration, notamment micro-structuration, de surfaces, modifications des régimes thermo-hydrauliques, entre autres.

## Revendications

1. Réacteur tubulaire (1) à lit fixe qui s'étend, selon un axe longitudinal (XX'), entre une première extrémité (11) et une deuxième extrémité (12), ledit réacteur (1) comprenant un lit de poudre catalytique confiné dans un espace annulaire (30) situé entre une paroi externe (15) d'un tube creux (10) et une paroi interne (21) d'un insert creux (20) disposé de manière coaxiale dans le tube creux (10), l'insert creux (20) comprenant au moins une chambre de distribution (40) et au moins une chambre de collecte (50), séparées l'une de l'autre par au moins une première paroi séparatrice (60), ladite au moins une chambre de distribution (40) et ladite au moins une chambre de collecte (50) comprenant, respectivement, une ouverture d'admission (42) de gaz au niveau de la première extrémité (11) et une ouverture d'évacuation (51) de gaz au niveau de la deuxième extrémité (12),

   **caractérisé en ce que** la paroi interne (21) de l'insert creux (20) est recouverte d'une structure séparatrice (160, 170) comprenant au moins une membrane permsélective (160) permettant une élimination partielle d'au moins un produit de réaction ($H_2O$), de sorte que l'espace annulaire (30) soit délimité par la paroi externe (15) et la membrane permsélective (160),
   et **en ce que** le réacteur (1) comporte en plus au moins une chambre d'appoint (100) séparée de ladite au moins une chambre de distribution (40) et de ladite au moins une chambre de collecte (50) par au moins une première paroi séparatrice (60), ladite au moins une chambre d'appoint (100) comprenant un orifice d'entrée (110) d'au moins un fluide d'appoint (F'), constitué par un fluide de balayage (F') permettant l'évacuation dudit au moins un produit de réaction ($H_2O$), distinct des gaz circulant dans ladite au moins une chambre de distribution (40) et dans ladite au moins une chambre de collecte (50), au niveau de la première extrémité (11) et un orifice de sortie (112) dudit au moins un fluide d'appoint (F') au niveau de la deuxième extrémité (12).

2. Réacteur selon la revendication 1, **caractérisé en ce que** la perméabilité de la membrane permsélective (160) s'exerce de façon sélective vis-à-vis de la vapeur d'eau ($H_2O$).

3. Réacteur selon la revendication 1 ou 2, **caractérisé en ce que** la structure séparatrice (160, 170) comporte de plus un support poreux (170) recouvrant la paroi interne (21) de l'insert (20), le support poreux (170) étant lui-même recouvert par la membrane permsélective (160), et **en ce que** l'insert creux (20) comporte des perforations (180) pour permettre le passage dudit au moins un produit de réaction ($H_2O$) à séparer desdits gaz.

4. Réacteur selon la revendication 1 ou 2, **caractérisé en ce que** l'insert creux (20) est réalisé en un matériau poreux, pour permettre le passage dudit au moins un produit de réaction ($H_2O$) à séparer desdits gaz, et recouvert par la membrane permsélective (160), et **en ce que** ladite au moins une première paroi séparatrice (60) de l'insert creux (20) comporte un matériau d'étanchéité, notamment céramique, pour l'étanchéité vis-à-vis des gaz réactifs.

5. Réacteur selon l'une quelconque des revendications précédentes, **caractérisé en ce qu'**il comporte au niveau de la première extrémité (11) et au niveau de la deuxième extrémité (12), respectivement, un espace distributeur (42) et un espace collecteur (51) entre lesquels l'insert creux (20) est disposé.

6. Réacteur selon l'une quelconque des revendications précédentes, **caractérisé en ce que** le tube (10) et l'insert (20) sont maintenus par au moins deux plaques tubulaires de maintien (33) respectivement au niveau des première (11) et deuxième (12) extrémités, au moins l'une desdites au moins deux plaques tubulaires de maintien (33), notamment toutes les plaques tubulaires de maintien (33), comportant un conduit d'entrée ou de sortie (111, 113) respectivement relié fluidiquement à un orifice d'entrée (110) ou de sortie (112) de fluide d'appoint (F') et formé dans ladite au moins l'une desdites au moins deux plaques tubulaires de maintien (33), pour permettre notamment une admission et/ou une extraction latérale de fluide d'appoint (F').

7. Réacteur selon la revendication 5, **caractérisé en ce que** le tube (10) et l'insert (20) sont maintenus par au moins deux plaques tubulaires de maintien (33) respectivement au niveau des première (11) et deuxième (12) extrémités, et **en ce qu'**au moins un conduit d'entrée ou de sortie (111, 113) respectivement relié fluidiquement à un orifice d'entrée (110) ou de sortie (112) de fluide d'appoint (F') est situé respectivement dans l'espace distributeur (42) et dans l'espace collecteur (51), hors desdites au moins deux plaques tubulaires de maintien (33), pour permettre notamment une admission et/ou une extraction latérale de fluide d'appoint (F').

8. Réacteur selon la revendication 5, **caractérisé en ce que** le tube (10) et l'insert (20) sont maintenus par au moins deux plaques tubulaires de maintien (33) respectivement au niveau des première (11) et deuxième (12) extrémités, et en ce ledit orifice d'entrée (110) et ledit orifice de sortie (113) dudit au moins un fluide d'appoint (F') sont respectivement formés dans les extrémités supérieure et inférieure de l'insert (20) pour permettre respectivement une alimentation depuis l'espace distributeur (42) et une extraction depuis l'espace collecteur (51), le réacteur (1) comprenant en outre au moins un conduit d'alimentation latéral (140) de gaz et au moins un conduit d'extraction latéral (141) de gaz, ledit au moins un conduit d'alimentation latéral (140) et l'alimentation en fluide d'appoint (F') de l'espace distributeur (42) d'une part, et ledit au moins un conduit d'extraction latéral (141) et l'extraction en fluide d'appoint (F') de l'espace collecteur (51) d'autre part, étant notamment séparés par une plaque de séparation étanche (105).

9. Réacteur selon la revendication 8, **caractérisé en ce qu'**un joint d'étanchéité (106) est disposé entre l'insert (20) et chacune des plaques de séparation étanches (105).

10. Réacteur selon l'une quelconque des revendications précédentes, **caractérisé en ce que** l'écoulement dudit au moins un fluide d'appoint (F') est réalisé de manière co-courante ou contre-courante à l'écoulement des gaz dans lesdites au moins une chambre de distribution (40) et de collecte (50).

11. Réacteur selon l'une quelconque des revendications précédentes, **caractérisé en ce que** la poudre catalytique est retenue dans l'espace annulaire (30) par un joint (31) en matière fibreuse au niveau de chacune des extrémités de l'espace annulaire (30).

12. Réacteur selon l'une quelconque des revendications précédentes, **caractérisé en ce que** l'insert (20) est une pièce monobloc.

13. Utilisation d'un réacteur tubulaire (1) selon l'une quelconque des revendications précédentes, **caractérisée en ce qu'**elle met en œuvre des réactions endothermiques ou exothermiques de synthèse de carburants et de combustibles.

**Patentansprüche**

1. Rohrreaktor (1) mit Festbett, der sich entlang einer Längsachse (XX') zwischen einem ersten Ende (11) und einem zweiten Ende (12) erstreckt, wobei der Reaktor (1) ein Bett aus Katalysatorpulver umfasst, das in einem Ringraum (30) eingeschlossen ist, der sich zwischen einer Außenwand (15) eines Hohlrohrs (10) und einer Innenwand (21) eines Hohleinsatzes (20) befindet, der koaxial im Hohlrohr (10) angeordnet ist, wobei der Hohleinsatz (20) mindestens eine Verteilerkammer (40) und mindestens eine Sammelkammer (50) umfasst, die durch mindestens eine erste Trennwand (60) voneinander getrennt sind, wobei die mindestens eine Verteilerkammer (40) und die mindestens eine Sammelkammer (50) jeweils eine Gaszufuhröffnung (42) an dem ersten Ende (11) und eine Gasablassöffnung (51) an dem zweiten Ende (12) umfassen,

   **dadurch gekennzeichnet, dass** die Innenwand (21) des Hohleinsatzes (20) mit einer Trennstruktur (160, 170) bedeckt ist, die mindestens eine permselektive Membran (160) umfasst, die eine teilweise Entfernung von mindestens einem Reaktionsprodukt ($H_2O$) ermöglicht, so dass der Ringraum (30) durch die Außenwand (15) und die permselektive Membran (160) begrenzt ist,
   und dass der Reaktor (1) ferner mindestens eine Zusatzkammer (100) aufweist, die von der mindestens einen Verteilerkammer (40) und von der mindestens einen Sammelkammer (50) durch mindestens eine erste Trennwand (60) getrennt ist, wobei die mindestens eine Zusatzkammer (100) eine Einlassöffnung (110) für mindestens ein Zusatzfluid (F'), das aus einem Spülfluid (F') besteht, das das Ablassen des mindestens einen Reaktionsprodukts ($H_2O$) ermöglicht, das sich von den Gasen, die in der mindestens einen Verteilerkammer (40) und in der mindestens einen Sammelkammer (50) zirkulieren, unterscheidet, an dem ersten Ende (11) und eine Auslassöffnung (112) für das mindestens eine Zusatzfluid (F') an dem zweiten Ende (12) umfasst.

2. Reaktor nach Anspruch 1, **dadurch gekennzeichnet, dass** die Permeabilität der permselektiven Membran (160) selektiv gegenüber Wasserdampf ($H_2O$) stattfindet.

3. Reaktor nach Anspruch 1 oder 2, **dadurch gekennzeichnet, dass** die Trennstruktur (160, 170) ferner einen porösen Träger (170) aufweist, der die Innenwand (21) des Einsatzes (20) bedeckt, wobei der poröse Träger (170) selbst von der permselektiven Membran (160) bedeckt ist, und dass der Hohleinsatz (20) Perforationen (180) aufweist, um den Durchgang des mindestens einen von den Gasen zu trennenden Reaktionsprodukts ($H_2O$) zu ermöglichen.

4. Reaktor nach Anspruch 1 oder 2, **dadurch gekennzeichnet, dass** der Hohleinsatz (20) aus einem porösen Material hergestellt ist, um den Durchgang des mindestens einen von den Gasen zu trennenden Reaktionsprodukts ($H_2O$) zu ermöglichen, und von der permselektiven Membran (160) bedeckt ist, und dass die mindestens eine erste Trennwand (60) des Hohleinsatzes (20) ein Dichtungsmaterial, insbesondere Keramik, zur Abdichtung gegenüber den reaktiven Gasen aufweist.

5. Reaktor nach einem der vorhergehenden Ansprüche, **dadurch gekennzeichnet, dass** er an dem ersten Ende (11) und an dem zweiten Ende (12) jeweils einen Verteilerraum (42) und einen Sammelraum (51) aufweist, zwischen denen der Hohleinsatz (20) angeordnet ist.

6. Reaktor nach einem der vorhergehenden Ansprüche, **dadurch gekennzeichnet, dass** das Rohr (10) und der Einsatz (20) durch mindestens zwei Halterohrplatten (33) jeweils an dem ersten (11) und dem zweiten (12) Ende gehalten werden, wobei mindestens eine der mindestens zwei Halterohrplatten (33), insbesondere alle Halterohrplatten (33), einen Einlass- oder einen Auslasskanal (111, 113) aufweisen, der jeweils fluidisch mit einer Einlass- (110) oder einer Auslassöffnung (112) für Zusatzfluid (F') verbunden ist und in der mindestens einen der mindestens zwei Halterohrplatten (33) ausgebildet ist, um insbesondere eine seitliche Zufuhr und/oder eine seitliche Entnahme von Zusatzfluid (F') zu ermöglichen.

7. Reaktor nach Anspruch 5, **dadurch gekennzeichnet, dass** das Rohr (10) und der Einsatz (20) durch mindestens zwei Halterohrplatten (33) jeweils an dem ersten (11) und dem zweiten (12) Ende gehalten werden, und dass mindestens ein Einlass- oder ein Auslasskanal (111, 113), der jeweils fluidisch mit einer Einlass-(110) oder einer Auslassöffnung (112) für Zusatzfluid (F') verbunden ist, sich jeweils im Verteilerraum (42) und im Sammelraum (51) befindet, außerhalb der mindestens zwei Halterohrplatten (33), um insbesondere eine seitliche Zufuhr und/oder eine seitliche Entnahme von Zusatzfluid (F') zu ermöglichen.

8. Reaktor nach Anspruch 5, **dadurch gekennzeichnet, dass** das Rohr (10) und der Einsatz (20) durch mindestens zwei Halterohrplatten (33) jeweils an dem ersten (11) und dem zweiten (12) Ende gehalten werden, und dass die Einlassöffnung (110) und die Auslassöffnung (113) für das mindestens eine Zusatzfluid (F') jeweils in dem oberen und dem unteren Ende des Einsatzes (20) ausgebildet sind, um eine Versorgung aus dem Verteilerraum (42) und eine Entnahme aus dem Sammelraum (51) zu ermöglichen, wobei der Reaktor (1) ferner mindestens einen seitlichen Gasversorgungskanal (140) und mindestens einen seitlichen Gasentnahmekanal (141) umfasst, wobei der mindestens eine seitliche Versorgungskanal (140) und die Versorgung des Verteilerraums (42) mit Zusatzfluid (F') einerseits und der mindestens eine seitliche Entnahmekanal (141) und die Entnahme von Zusatzfluid (F') aus dem Sammelraum (51) andererseits, insbesondere durch eine wasserdichte Trennplatte (105), voneinander getrennt sind.

9. Reaktor nach Anspruch 8, **dadurch gekennzeichnet, dass** zwischen dem Einsatz (20) und jeder der wasserdichten Trennplatten (105) eine Dichtung (106) angeordnet ist.

10. Reaktor nach einem der vorhergehenden Ansprüche, **dadurch gekennzeichnet, dass** die Strömung des mindestens einen Zusatzfluids (F') im Gleichstrom oder Gegenstrom zur Strömung der Gase in der mindestens einen Verteiler- (40) und einen Sammelkammer (50) erfolgt.

11. Reaktor nach einem der vorhergehenden Ansprüche, **dadurch gekennzeichnet, dass** das Katalysatorpulver im Ringraum (30) durch eine Dichtung (31) aus Faserstoff an jedem der Enden des Ringraums (30) zurückgehalten wird.

12. Reaktor nach einem der vorhergehenden Ansprüche, **dadurch gekennzeichnet, dass** der Einsatz (20) ein einteiliges Bauteil ist.

13. Verwendung eines Rohrreaktors (1) nach einem der vorhergehenden Ansprüche, **dadurch gekennzeichnet, dass** er endotherme oder exotherme Reaktionen zur Synthese von Kraftstoffen und Brennstoffen umsetzt.

**Claims**

1. A fixed-bed tubular reactor (1) which extends, along a longitudinal axis (XX'), between a first end (11) and a second end (12), said reactor (1) comprising a catalytic powder bed confined in an annular space (30) situated between an outer wall (15) of a hollow tube (10) and an inner wall (21) of a hollow insert (20) disposed coaxially in the hollow tube (10), the hollow insert (20) comprising at least one distribution chamber (40) and at least one collection chamber (50), separated from each other by at least one first separative wall (60), said at least one distribution chamber (40) and said at least one collection chamber (50) comprising a gas intake opening (42) at the first end (11) and a gas discharge opening (51) at the second end (12) respectively,

   **characterised in that** the inner wall (21) of the hollow insert (20) is covered with a separative structure (160, 170) comprising at least one permselective membrane (160) for partially removing at least one reaction product ($H_2O$), so that the annular space (30) is delimited by the outer wall (15) and the permselective membrane (160), and **in that** the reactor (1) further includes at least one supplemental chamber (100) separated from said at least one distribution chamber (40) and from said at least one collection chamber (50) by at least one first separative wall (60), said at least one supplemental chamber (100) comprising an inlet port (110) for at least one supplemental fluid (F'), consisting of a flushing fluid (F') for discharging said at least one reaction product ($H_2O$), distinct from the gases circulating in said at least one distribution chamber (40) and in said at least one collection chamber (50), at the first end (11), and an outlet port (112) for said at least one supplemental fluid (F'), at the second end (12).

2. The reactor according to claim 1, **characterised in that** the permeability of the permselective membrane (160) is selectively exerted with respect to water vapour ($H_2O$).

3. The reactor according to claim 1 or 2, **characterised in that** the separative structure (160, 170) additionally includes a porous support (170) covering the inner wall (21) of the insert (20), the porous support (170) itself being covered with the permselective membrane (160), and **in that** the hollow insert (20) includes apertures (180) to allow said at least one reaction product ($H_2O$) to be separated from said gases to pass therethrough.

4. The reactor according to claim 1 or 2, **characterized in that** the hollow insert (20) is made of a porous material, to allow said at least one reaction product ($H_2O$) to be separated from said gases to pass therethrough, and is covered with the permselective membrane (160), and **in that** said at least one first separative wall (60) of the hollow insert (20) includes a sealing, especially ceramic, material, for sealing with respect to the reactive gases.

5. The reactor according to any of the preceding claims, **characterised in that** it includes at the first end (11) and at the second end (12), respectively, a distributing space (42) and a collecting space (51) between which the hollow insert (20) is disposed.

6. The reactor according to any of the preceding claims, **characterised in that** the tube (10) and the insert (20) are held by at least two tubular holding plates (33) at the first (11) and second (12) ends respectively, at least one of said at least two tubular holding plates (33), especially all the tubular holding plates (33), including an inlet or outlet duct (111, 113) respectively fluidically connected to an inlet port (110) or outlet port (112) for supplemental fluid (F') and formed in said at least one of said at least two tubular holding plates (33), especially to allow side intake and/or extraction of supplemental fluid (F').

7. The reactor according to claim 5, **characterised in that** the tube (10) and the insert (20) are held by at least two tubular holding plates (33) respectively at the first (11) and second (12) ends, and **in that** at least one inlet or outlet duct (111, 113) respectively fluidically connected to an inlet port (110) or outlet port (112) for supplemental fluid (F') is situated in the distributing space (42) and in the collecting space (51) respectively, outside said at least two tubular holding plates (33), especially to allow side intake and/or extraction of supplemental fluid (F').

8. The reactor according to claim 5, **characterised in that** the tube (10) and the insert (20) are held by at least two tubular holding plates (33) at the first (11) and second (12) ends respectively, and **in that** said inlet port (110) and said outlet port (113) for said at least one supplemental fluid (F') are respectively formed in the upper and lower ends of the insert (20) for feeding from the distributing space (42) and extracting from the collecting space (51) respectively, the reactor (1) further comprising at least one side gas feed duct (140) and at least one side gas extraction duct (141), said at least one side feed duct (140) and the feed of fluid supplemental (F') from the distributing space (42) on the one hand, and said at least one side extraction duct (141) and the extraction of supplemental fluid (F') from the collecting space (51) on the other hand, being especially separated by a sealed separation plate (105).

9.  The reactor according to claim 8, **characterised in that** a seal (106) is disposed between the insert (20) and each of the sealed separation plates (105).

10. The reactor according to any of the preceding claims, **characterised in that** the flow of said at least one supplemental fluid (F') is made co-currently or countercurrently to the flow of gases in said at least one distribution (40) and collection (50) chamber.

11. The reactor according to any of the preceding claims, **characterised in that** the catalytic powder is retained in the annular space (30) by a seal (31) of fibrous material at each of the ends of the annular space (30).

12. The reactor according to any of the preceding claims, **characterised in that** the insert (20) is a one-piece part.

13. A use of a tubular reactor (1) according to any of the preceding claims, **characterised in that** it implements endothermic or exothermic reactions for the synthesis of fuels and combustibles.

FIG. 1

FIG. 2

FIG. 2B

FIG. 2A

EP 4 412 756 B1

FIG. 3

FIG. 4

FIG. 6

FIG. 5

EP 4 412 756 B1

FIG. 8

FIG. 7

EP 4 412 756 B1

FIG. 9

FIG. 10

FIG. 11

FIG. 12

FIG. 13

FIG.14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3758279 A
- US 4374094 A
- EP 0560157 A1
- US 2997374 A
- CN 103990420 A
- US 8961909 A
- US 7402719 B2
- FR 3103714 A1

**Littérature non-brevet citée dans la description**

- In-Situ H2O removal via hydrophilic membranes during Fischer-Tropsch and other fuel-related synthesis reactions. **M.P.RHODE**. Phd Dissertation. KIT Scientific Publishing, 2010